(19) **Europäisches Patentamt / European Patent Office / Office européen des brevets**

(11) **EP 4 019 061 B1**

(12) **EUROPEAN PATENT SPECIFICATION**

(45) Date of publication and mention
of the grant of the patent:
**11.06.2025 Bulletin 2025/24**

(21) Application number: **20216613.8**

(22) Date of filing: **22.12.2020**

(51) International Patent Classification (IPC):
**A61M 1/28** (2006.01)    **A61M 1/16** (2006.01)
**A61M 5/44** (2006.01)    **H05B 3/28** (2006.01)
**H05B 3/36** (2006.01)

(52) Cooperative Patent Classification (CPC):
**A61M 1/287; A61M 1/166; H05B 3/36;** A61M 5/445;
A61M 2205/3653; H05B 2203/021; H05B 2203/022

(54) **SILICONE RUBBER HEATER FOR PERITONEAL DIALYSIS**

SILIKONGUMMIERHITZER FÜR PERITONEALDIALYSE

CHAUFFAGE DE CAOUTCHOUC DE SILICONE POUR DIALYSE PÉRITONÉALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**29.06.2022 Bulletin 2022/26**

(73) Proprietor: **Bellco S.r.l.**
**41037 Mirandola (IT)**

(72) Inventor: **MARRA, Antonio Giuseppe**
**Mirandola (IT)**

(74) Representative: **Maschio & Soames IP Ltd**
**30 Carlton Crescent**
**Southampton SO15 2EW (GB)**

(56) References cited:
WO-A1-2019/112548    US-A- 5 932 110
US-A1- 2003 000 939    US-A1- 2004 060 865
US-A1- 2006 065 276    US-A1- 2014 199 057
US-A1- 2020 261 638

**Description**

FIELD

The disclosure relates to a heater made from a layer of silicone rubber or similar material having one or more embedded heating elements and a conductive plate such as an aluminum plate positioned on a side of the layer of silicone rubber. The heater having a layer of silicone rubber with a corresponding conductive plate can be shaped into many different sizes and shapes to uniformly heat a fluid receptacle of any size or shape in any heating compartment wherein the compartment also can have any size of shape. The heater can be used in any application where a fluid must be heated uniformly in a receptacle of any size of placement. The heater can be used to heat fluids necessary to generate a peritoneal dialysis fluid or heat peritoneal dialysis fluid prior to infusion into a patient. Systems that have one or more of the heaters of the invention to generate the peritoneal dialysis fluid and to heat the generated peritoneal dialysis fluid are provided. An example of a prior art device is disclosed in US2014/0199057 A1.

BACKGROUND

Heaters often fail to uniformly or accurately heat fluids to a specific temperature. The problem can be aggravated where the heater is required to heat fluids or water in fluid receptacles of different sizes and shapes. Heaters are often made from inflexible materials such as ceramic and cannot be easily engineered into tight spaces, unusually shaped designs, or specific configurations to uniformly heat fluid receptacles or containers of various shapes and sizes. Yet accurate and precise heating control is often required in many applications where heat is delivered to fluid in order to reach or attain a specific fluid temperature. The known heaters and systems often fail to heat fluids to a specific temperature due to the creation of hot or cold spots on the surface of the heater that translate into non-uniform heating of a fluid receptacle or container. The known heaters often fail to provide uniform heating because they contain hot or cold spots. Because of the high heating power density of certain heaters, such as ceramic heaters, the fluids in the bags or containers do not heat uniformly. This can cause problems in applications where a specific temperature is required to dissolve or mix solutions or solutes such as during peritoneal dialysis. Non-uniform heating of the fluid also introduces errors into temperature measurements, as the temperature measured becomes dependent upon the location of the sensor in the container or bag. This can pose problems for creating a medical solution that requires precise and accurate temperature control to dissolve certain constituents at a particular fluid temperature.

The known heaters also sometimes fail to uniformly heat peritoneal dialysis fluid that is delivered to patients. Notably, peritoneal dialysis must be heated to body temperature prior to infusion for the health, safety, and comfort of the patient. Existing systems such as cyclers having a heater cannot heat fluid receptacles or containers of various shapes or sizes. The existing systems often are configured as a flat plate onto which a receptacle must rest. This can cause uneven heating and create a burn hazard. Known devices often fail to account for home use and cannot be engineered to size, shape, and geometry constraints.

Hence, there is a need for systems and methods that can heat fluid in a bag or container accurately, precisely, or uniformly. The need includes systems and methods that use a lower heating power density heater to more uniformly heat the fluid, improving temperature measurement accuracy and/or precision. The need extends to a heater that can be designed and fabricated into various shapes, sizes, and geometries to accommodate fluid bags or containers of various shapes, sizes, and geometries. The need extends to heaters that can fit inside various receiving compartments or spaces insides devices. The need extends further to heaters that can be engineered into tight spaces, unusually shaped designs, or specific configurations. The need includes accurate and precise heating control so that a fluid container being heated by the heater uniformly reaches or attains a specific fluid temperature quickly and accurately. The need includes further systems that can heat fluids to a specific temperature without overheating or underheating specific spots on a fluid receptacle or container.

SUMMARY OF THE INVENTION

The problem to be solved by the present invention is accurate and uniform heating of fluids used in generating fluid to dissolve concentrates for use in peritoneal dialysis fluid and/or in heating a peritoneal dialysis fluid to a suitable temperature for treating a patient with peritoneal dialysis fluid. The solution can use a heater that has a low heating power density made from a flexible material layer such as silicone rubber having heating elements embedded therein, that is affixed or adhered to a conductive plate such as an aluminum plate to uniformly heat a fluid in a bag. The invention is defined by the appended claims.

## BRIEF DESCRIPTION OF THE DRAWINGS

FIG.'s 1A-D illustrate a peritoneal dialysis system having a substantially flat silicone rubber heater.

FIG.'s 2A-D illustrate a peritoneal dialysis system having a substantially rectangular receiving compartment for heating a fluid bag.

FIG. 3 illustrates a peritoneal dialysis system having a substantially rectangular receiving compartment with a lid for heating a fluid bag.

FIG.'s 4A-D illustrate a peritoneal dialysis system having a triangular receiving compartment for heating a fluid bag.

FIG. 5 illustrates a peritoneal dialysis system having a triangular receiving compartment with a lid for heating a fluid bag.

FIG.'s 6A-D illustrate a peritoneal dialysis system having a substantially round receiving compartment for heating a fluid bag.

FIG. 7 illustrates a peritoneal dialysis system having a substantially round receiving compartment with a lid for heating a fluid bag.

FIG.'s 8A-D illustrate a peritoneal dialysis system having a hexagonal receiving compartment for heating a fluid bag.

FIG. 9 illustrates a peritoneal dialysis system having a hexagonal receiving compartment with a lid for heating a fluid bag.

FIG. 10 illustrates a peritoneal dialysis system including a cycler and generation module each using a silicone rubber heater.

FIG. 11 is a flow diagram of a peritoneal dialysis generation module.

FIG. 12 is an experimental setup used to test a silicone rubber heater.

FIG.'s 13A-C are graphs of temperature vs. time for heating fluid in a bag with a silicone rubber heater.

FIG.'s 14A-C are images from an experiment to determine the uniformity of temperature using a silicone rubber heater.

## DETAILED DESCRIPTION

Unless defined otherwise, all technical and scientific terms used have the same meaning as commonly understood by one of ordinary skill in the art.

The articles "a" and "an" are used to refer to one to over one (i.e., to at least one) of the grammatical object of the article. For example, "an element" means one element or over one element.

An "aluminum plate" can be a piece of aluminum metal including aluminum alloys containing any percentage of other suitable metal or material known to those of skill in the art for appropriate thermal conductivity. The piece can be of any planar shape, size, or thickness.

The term "comprising" includes, but is not limited to, whatever follows the word "comprising." Use of the term indicates the listed elements are required or mandatory but that other elements are optional and may be present.

The term "consisting of" includes and is limited to whatever follows the phrase "consisting of." The phrase indicates the limited elements are required or mandatory and that no other elements may be present.

The term "consisting essentially of" includes whatever follows the term "consisting essentially of" and additional elements, structures, acts, or features that do not affect the basic operation of the apparatus, structure or method described.

The term "concave" refers to a shape of a material that is curved inwardly.

A "concentrate" refers to a solution of solutes in water; the solution having a higher concentration than intended for use in treatment.

A "concentrate source" refers to a container from which a concentrate can be obtained.

A "copper plate" can be a piece of copper metal including copper alloys containing any percentage of other suitable metal or material known to those of skill in the art for appropriate thermal conductivity. The piece can be of any planar shape, size, or thickness.

The term "embedded" refers to a first component being fixed within completely or partially a second component or material.

To "generate" a fluid refers to a process of creating the fluid from constituent parts.

A "gold plate" can be a piece of gold metal including gold alloys containing any percentage of other suitable metal or material known to those of skill in the art for appropriate thermal conductivity. The piece can be of any planar shape, size, or thickness.

A "graphite plate" can be a piece of graphite including graphite composites containing any percentage of other suitable metal or material known to those of skill in the art for appropriate thermal conductivity. The piece can be of any planar shape, size, or thickness.

A "heater" is a component that is used to raise the temperature of container or substance.

The terms "heating" or to "heat" refer to a process of raising a temperature of a substance or container.

A "heating element" is an electrical component that increases in temperature as an electrical current flows through the component.

To "infuse" a fluid refers to the process of flowing a fluid into a cavity.

An "inner surface" of a component refers to a wall of the component that is inside of the component or system.

The term "initially" refers to a state of a component or system prior to a process.

A "layer of silicone rubber" refers to an elastomeric material composed of a silicone-based polymer of any suitable type known to those of skill in the art.

The term "lining" or to "line" refers to a first material or component covering a surface of a second material or component.

The term "on top" refers to the relative position of two components wherein a first component is positioned above a second component when arranged for normal use. The first component is on top of the second component.

A "peritoneal dialysis cycler" is a component or set of components for movement of fluid into and out of the peritoneal cavity of a patient.

"Peritoneal dialysis fluid" is a dialysis solution to be used in peritoneal dialysis having specified parameters for purity and sterility. Peritoneal dialysis fluid is generally not the same as dialysate used in hemodialysis.

A "fluid bag" is a bag or container that contains any fluid or aqueous solution. One type of "fluid bag" is a "peritoneal dialysis fluid bag" that can contain either peritoneal dialysis fluid or any fluid used in generating peritoneal dialysis fluid.

A "peritoneal dialysis fluid generation module" is a component or set of components for generating peritoneal dialysis fluid from one or more solid substances, concentrates, or solutions containing components of peritoneal dialysis fluid.

The term "polydimethylsiloxane" also known as dimethylpolysiloxane or dimethicone, refers to a group of polymeric organosilicon compounds that are commonly referred to as silicones.

The term "polysiloxane" refers to any polymer having an inorganic backbone of - (Si-O)- repeat units.

The term "position" or "positioned" refers to a physical location of a component or system.

The terms "pumping" or to "pump" refer to moving a fluid or gas using suction or pressure.

The term "purified water" refers to water that has been treated to remove chemical and/or biological contaminants.

A "receiving compartment" can be a compartment, section, or chamber within a larger system into which a component can be positioned.

The term "silicone rubber" refers to any elastomer containing silicon together with carbon, hydrogen, and oxygen. Silicone rubbers include any type, form, or composition of polysiloxanes or polydimethylsiloxanes. The term can also refer to any polymer having a Si-O-Si backbone. Any grade or form of silicone rubber is contemplated that is suitable for the intended use.

A "silicone carbide plate" can be a piece of silicone carbide including silicone carbide composites containing any percentage of other suitable metal or material known to those of skill in the art for appropriate thermal conductivity. The piece can be of any planar shape, size, or thickness.

The terms "solid" or "solid material" refer to a material in the solid phase of matter, and can include crystalline, powdered, or any other form of solid material.

The term "substantially flat" refers generally to the shape of a component that is smooth and even throughout. However, a "substantially flat" component can include slight variations in elevations or include small protuberances.

The term "substantially rectangular" refers generally to a three-dimensional shape of a component that has six quadrilateral faces, with opposite faces having the same dimensions. However, the actual contours of a "substantially rectangular" component can vary slightly from a perfect rectangular prism.

The term "substantially round" refers generally to the shape of a sphere or portion of a sphere. However, the actual contours of a "substantially round" component can vary slightly from a perfect spheroid.

The term "substantially similar to an area" refers to a first component having a surface area that generally has the same are as a second referenced component.

The term "thermally conductive metal" refers to any metal capable of conducting thermal heat such as copper, aluminum, brass, steel, bronze, and other similar materials known to those of skill in the art.

The term "thermally conductive metal alloy" refers to any metal combination or metal alloy that contains any percent combination of metals capable of conducting thermal heat such as copper, aluminum, brass, steel, bronze, and other similar materials known to those of skill in the art. The metal alloy can contain any suitable type of constituent such as carbon, silica, chemical additives, or elements such as lithium, sodium, or calcium. The list is non-exhaustive and includes any suitable alloy known to those of skill in the art that is a suitable heat conductor.

The term "thermally conductive composite" refers to any non-metal base material that can be used to conduct heat. For example, fibrous carbon material can be compounded in a metal matrix powder of aluminum or the like to fabricate a thermally conductive composite material. In other examples, the composite can be a conductive molded composition made from a polymer base matrix and the like. Other suitable composites known to those of skill in the art are contemplated.

A "tungsten plate" can be a piece of tungsten including tungsten composites containing any percentage of other suitable metal or material known to those of skill in the art for appropriate thermal conductivity. The piece can be of any planar shape, size, or thickness.

The term "uniformly distributed" refers to an arrangement of components embedded in a material; the arrangement substantially the same distance of the components distributed throughout the material. The arrangement can be described by any suitable configuration, geometry, or density of the component being distributed.

A "zinc plate" can be a piece of zinc including zinc composites containing any percentage of other suitable metal or material known to those of skill in the art for appropriate thermal conductivity. The piece can be of any planar shape, size, or thickness.

*Heater*

The heater and related systems and methods can accurately and/or precisely heat water or fluid to a specific temperature. The heater can deliver uniform heating to accurately and/or precisely heat water or other fluids in a bag, container, or fluid receptacle made of various shapes, sizes, and materials. Specifically, the heater can be fashioned into any one or more combination of shapes and sizes that can be matched or lined to a standard device or customized to a particular configuration or design. The heaters can be used in any industrial, medical, or manufacturing process requiring precision and/or accurate heating of water or fluids. For example, the heaters can be used to heat fluid and water for chemical and plating methods, gas generation, solvent and deionized water methods, and medical applications.

In one embodiment, the heaters heat water and other fluids used to generate peritoneal dialysis. The heaters can be shaped and sized to fit inside or on top of peritoneal dialysis devices intended for home use. Often, home-use peritoneal dialysis devices must be compact or have a specific shape, geometry, or size. As such, the heaters can be fashioned to line a recessed compartment, cavity, niche, crevice, or surface of the device. The heaters can then uniformly heat water or fluid contained inside a bag shaped or sized to fit inside the heating compartment or on top of the heating surface. The uniformly heated fluid or water can then be used to dissolve and/or mix various constituents at a precise and/or accurate temperature that may be different for each constituent. The heaters of the present invention can also be used to heat any medical fluid, such as a peritoneal dialysis fluid, to a specific temperature, such as body temperature, prior to infusion into a patient. One or more heaters of the invention can be combined into a system performing one or more heating at various stages of fluid generation and therapy.

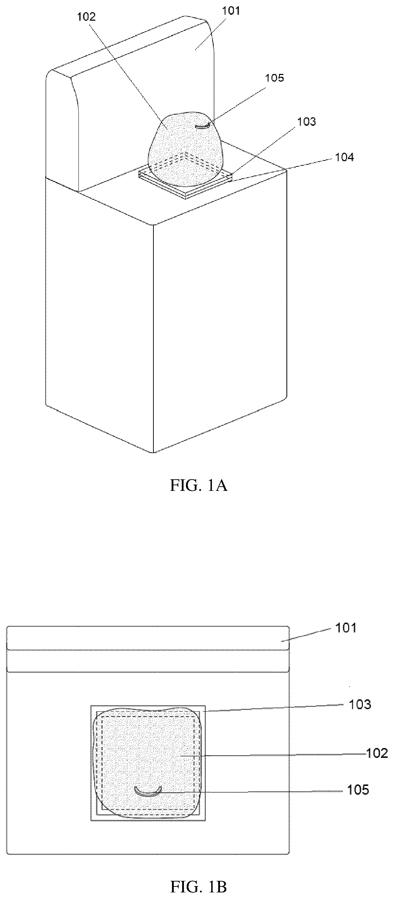

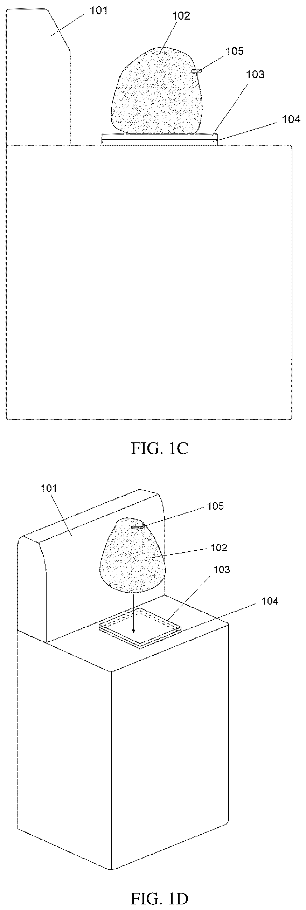

FIG.'s 1A-D illustrate a peritoneal dialysis system **101** using a heater. Although a peritoneal dialysis system is provided, any industrial, medical, or manufacturing process requiring precision and/or accurate heating of water or fluids is contemplated. FIG. 1A is a perspective view of the peritoneal dialysis system **101,** FIG. 1B is a top view of the peritoneal dialysis system **101,** FIG. 1C is a side view of peritoneal dialysis system **101,** and FIG. 1D shows a fluid bag **102** being placed onto a heater fabricated from a layer of a flexible thermally conductive material such as silicone rubber. In one embodiment, silicone rubber is contemplated and is described herein as a layer of silicone rubber **104** with embedded heating elements. The flexible conductive material can be any suitable material known to one of skill in the art having the desired properties and features capable of being embedded with heating elements. The thermally conductive silicone rubber compositions can be any thermally conductive silicones having suitable temperature and manufacturing properties. One nonlimiting group of thermally conductive silicone rubber compositions include those containing a polymer base matrix, organopolysiloxanes with vinyl groups or silica groups, organohydrogenpolysiloxanes, tackifiers selected from thermally conductive fillers, aminosilanes, epoxy silanes, and alkyl titanates. One of ordinary skill in the art will understand that any suitable silicone rubber or other flexible material having any one or more appropriate features relating to tensile strength, temperature resistance, molding, heat dissipation, melting, adhesion, and/or conductive properties can be used.

In the described embodiment, the layer of flexible, thermally conductive material such as a silicone rubber **104** is fixed or adhered to a conductive plate **103** positioned on one side of the layer of silicone rubber **104**. As illustrated in FIG. 1A, the heater can be positioned on a topside of a substantially flat surface of the peritoneal dialysis system **101**. As described herein, any industrial, medical, or manufacturing process requiring precision and/or accurate heating of water or fluids is contemplated. The heater can be electrically integrated into the peritoneal dialysis system **101** using any appropriate connection to deliver power to the heater. The heater can include a first layer of silicone rubber **104** and a conductive plate **103** positioned on one side of the layer of silicone rubber **104** such as a topside relative to the peritoneal dialysis system **101**. The layer of silicone rubber **104** can have heating elements (not shown) embedded within the silicone rubber **104**. In certain embodiments, the heating elements can be embedded completely inside, partially inside, or appurtenant to the silicone rubber **104**. The heating elements can be distributed in any suitable geometry or density to generate a desired watt density. Uniform or even distances between one or more heating element can encourage uniform heat distribution from the layer of silicone rubber 104 to the conductive plate **103**. The one or more heating elements are distributed through the silicone rubber **104** to provide substantially uniform radiant heating across the layer of silicone rubber **104**. One of ordinary skill can use any appropriate component or method necessary to deliver power or energy to the heating elements such as a wired, wireless, direct, magnetic, inductive, or any other connection or other suitable power delivery means. The heating elements can be made from nichrome, kanthal (FeCrAl) wire, cupronickel (CuNi), or etched foil, and those particularly having a low power density. Alternatively, any heating element can be arranged in a suitable density of an amount of wattage per square inch of surface area. For example, a heating element with 10 square inches of surface area, rated for 1,500 watts, can conduct 150 watts per square inch when in use. Similarly, the same 1,500 watt element can be uniformly distributed for 7.5 square inches of surface area to produce a higher density element, conducting 200 watts per square inch. One of ordinary skill will understand that various combinations, densities, and arrangements can be used to produce a desired watt density.

The conductive plate **103** can rest on a topside of the silicone rubber **104** to encourage uniform distribution of heat radiating from the first layer of silicone rubber **104** containing the embedded heating elements. The conductive plate **103** can be affixed or adhered to the silicone rubber **104** using glue, mechanical fixation, or any other suitable process or means. In combination with the heating elements uniformly embedded inside the layer of silicone rubber **104,** the conductive plate **103** can distribute heat in a uniform manner to an item to be heated. In one configuration, a fluid bag **102** can be positioned between the conductive plate **103** the silicone rubber **104.** The fluid bag **102** can contain water, peritoneal dialysis fluid, fluid concentrates, combinations thereof, or any other aqueous solution. The fluid bag **102** can be any container constructed from plastic, thermoplastic, fabric, or other suitable material that can be heated at the temperatures required for a particular application. For example, the fluid bag **102** can be constructed from materials including, but not limited to, polyethylene (PE), low density polyethylene (LDPE), linear low-density polyethylene (LLDPE), polypropylene (PP), ethyl vinyl acetate (EVA), polymers, copolymers, blockcopolymers, the like, and blends thereof. One of ordinary skill can make a blend of materials to produce a bag having desirable degrees of moldability, tensile strength, pliability, clarity, heat-resistance, heat-transmission, and the like.

The conductive plate **103** can be made from any thermally conductive material. For example, the thermally conductive material can be selected from aluminum, silver, copper, gold, silicone carbide, tungsten, graphite, zinc, and combinations thereof. In particular, the thermally conductive material can be fabricated from a thermally conductive metal, thermally conductive metal alloy, thermally conductive composite, or any combination thereof. The thermally conductive metal can be any metal capable of conducting thermal heat such as copper, aluminum, brass, steel, bronze, and other similar materials known to those of skill in the art. The thermally conductive metal alloy can be any combination of metals, chemicals, fillers, and elements containing any percent combination of the constituent components. For example, the thermally conductive metal alloy can be made from combinations of copper, aluminum, brass, steel, bronze, and other similar materials known to those of skill in the art, and include carbon, silica, chemical additives, or elements such as lithium, sodium, or calcium. The thermally conductive composite can be made from any non-metal base material used to conduct heat. For example, carbon material can be compounded in a metal matrix powder of aluminum or the like to fabricate a thermally conductive composite material. In other examples, the composite can be a conductive molded composition made from a polymer base matrix and the like. Other suitable composites known to those of skill in the art are contemplated.

The conductive plate **103** can be sized to be substantially cover an entire side of the layer of silicone rubber **104.** In this manner, the heat radiating from the uniformly embedded heating elements can, in turn, be transmitted uniformly through the thermally conductive material of the conductive plate **103**. The combination of uniform embedded heating elements and conductive plate can be sized to match the silicone rubber layer to encourage the uniform heating of the item to be heated. The heating elements can heat the silicone rubber **104,** which in turn heats the conductive plate **103** and the fluid bag **102**. A programable controller (not shown) in communication with a temperature sensor (not shown) can be programmed to control the electrical current to the heating elements to control heating of the fluid in the fluid bag **102** to a desired temperature. Optionally, the fluid bag **102** can include a handle **105** to assist in transporting the fluid bag **102.** In certain embodiments, the heater can have a single heating element embedded, molded, or drowned in the layer of silicone rubber **104.** The single heating element can be a serpentine shape, distributed throughout the layer of silicone rubber **104.** Alternatively, two or more separate serpentine heating elements can be drowned in the layer of silicone rubber **104.** Using two heating elements as a double silicone rubber heater can allow driving half power to each element, and may better control heating. Any number of heating elements can be included in the silicone rubber heater. In certain embodiments, the heating elements can be placed in the same plane, without overlapping of the heating elements in the silicone rubber **104** to provide better heating control. The heating elements can have a lower power density relative to ceramic heaters. Ceramic heaters generally have high heating power densities and smaller dimensions. The silicone rubber heater illustrated in FIG. 1 has a lower heating power density. A lower heating power density can improve temperature control. Because of the high density of a ceramic heater, the temperature of the fluid measured by a temperature sensor depends on the location of the sensor within the bag because the fluid heats unevenly, giving inaccurate readings. With the lower heating power density of the heating elements of the present invention, fluid within a fluid bag such as a peritoneal dialysis fluid bag, can be heated more uniformly, and provide more accurate temperature measurement and control.

As illustrated in FIG. 1B, the dialysis fluid bag **102** can rest on a first side of the conductive plate **103**. The first side can be a topside of the conductive plate **103**. Once placed on topside of a substantially flat surface, the peritoneal dialysis fluid bag **102** can be heated uniformly by the silicone rubber **104** wherein heat radiates through the conductive plate **103** and into the dialysis fluid bag **102**. As illustrated in FIG. 1C, the entirety of the fluid bag **102** can be positioned on a topside of conductive plate **103** and silicone rubber **104**. An area of the conductive plate **103** is substantially similar to an area of the first side of the first layer of silicone rubber **104**. As illustrated in FIG. 1D, the fluid bag **102** can be placed on top of the conductive plate **103** and silicone rubber **104** at the top of the peritoneal dialysis system **101**. However, other arrangements are contemplated. As illustrated in FIG.'s 1A-D, the silicone rubber **104** and conductive plate **103** can be substantially flat. However, because silicone rubber is flexible, in certain embodiments, the silicone rubber **104** and conductive plate **103** can be curved. For example, the silicone rubber **104** and conductive plate **103** can have a concave shape, with the fluid bag **102** fitting into the curve of the concave heater. Thus shape, the heater can be sized to match any surface, cavity, compartment, niche, or section of a device.

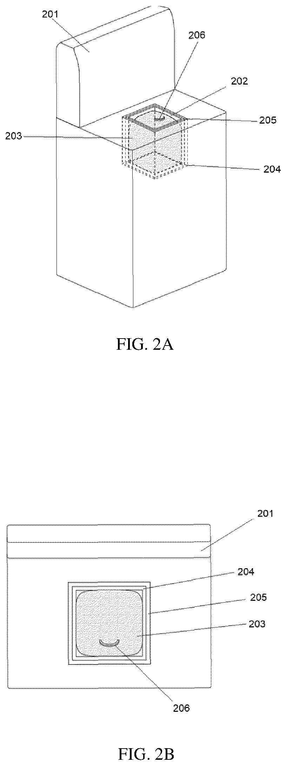

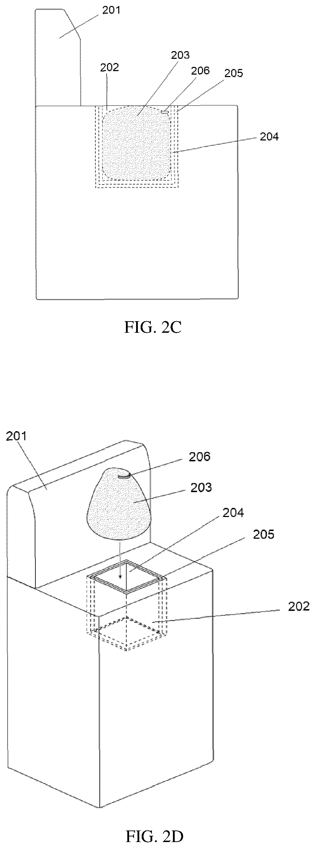

FIG.'s 2A-D illustrate a peritoneal dialysis system **201** with a silicone rubber heater lining a receiving compartment **202**. FIG.2A is a perspective view of the peritoneal dialysis system **201**, FIG. 2B is a top view of the peritoneal dialysis system **201**, FIG. 2C is a side view of peritoneal dialysis system **201**, and FIG. 2D shows a fluid bag **203** being placed into the receiving compartment **202**. As illustrated in FIG. 2A, the peritoneal dialysis system can include the receiving compartment **202** into which the fluid bag **203** is placed for heating. The receiving compartment in FIG.'s 2A-D can be rectangular; however, any shape can be used for the receiving compartment **202**. The heater can include a first layer of silicone rubber **205** and a conductive plate **204** positioned on top of the silicone rubber **205**. The layer of silicone rubber **205** has embedded heating elements to heat the silicone rubber **205**, which in turn heats the conductive plate **204**. The fluid bag **203** in contact with the conductive plate **204** is thus heated. Optionally, the fluid bag **203** can include a handle **206** to assist a user transport the fluid bag **203**. The receiving compartment **202** is lined with the silicone rubber **205** and conductive plate **204**, whereby the entire receiving compartment **202** can be used to heat the fluid bag **203** placed inside the receiving compartment **202**. In certain embodiments not forming part of the invention as claimed, the silicone rubber **205** and conductive plate **204** may not line the entire receiving compartment **202**, only lining a bottom portion receiving compartment **202**.

As illustrated in FIG. 2B, the fluid bag **203** fits entirely inside the receiving compartment **202** of the peritoneal dialysis system **201**. Once positioned inside the receiving compartment **202**, the fluid bag **203** can be heated by the silicone rubber **205** and conductive plate **204**. As illustrated in FIG. 2C, the fluid bag **203** can be placed fully within the receiving compartment **202**. The silicone rubber **205** and conductive plate **204** can line the inner surface of the receiving compartment **202**, contacting the fluid bag **203**. As illustrated in FIG. 2D, the fluid bag **203** can be loaded into the receiving compartment **202** from the top of the peritoneal dialysis system **201**. However, other arrangements are contemplated. For example, the receiving compartment **202** can be positioned in the front of the peritoneal dialysis system **201** and the fluid bag **203** can be loaded in from the front rather than the top. As described, handle **206** can be included on the fluid bag **203** for easier movement and loading into the receiving compartment **202**.

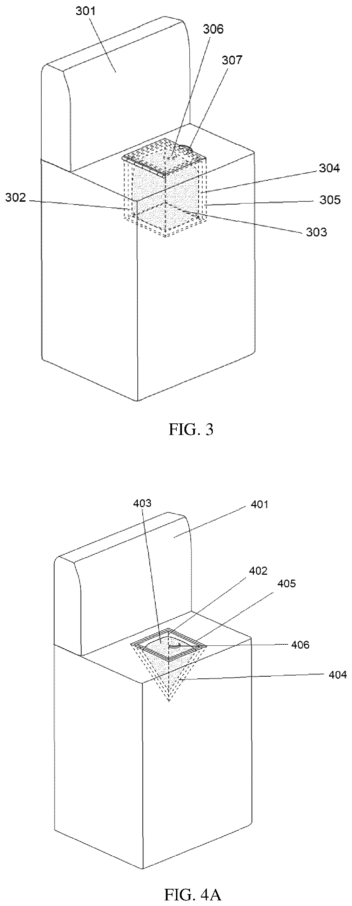

FIG. 3 illustrates a peritoneal dialysis system **301** having a rectangular receiving compartment **302**, similar to that shown in FIG.'s 2A-D. A layer of silicone rubber **305** and a conductive plate **304** can line all or part of an inner surface of receiving compartment **302** to heat fluid in fluid bag **303**. As described, the fluid bag **303** can include handle **306** for easier movement. The embodiment shown in FIG. 3 includes a lid **307** covering the receiving compartment **302**. The user can open lid **307**, load the fluid bag **303** into the receiving compartment **302**, and then close lid **307** to use the heater in a closed system to avoid heat loss and improved efficiency. The lid **307** can also be lined with a layer of silicone rubber and conductive plate.

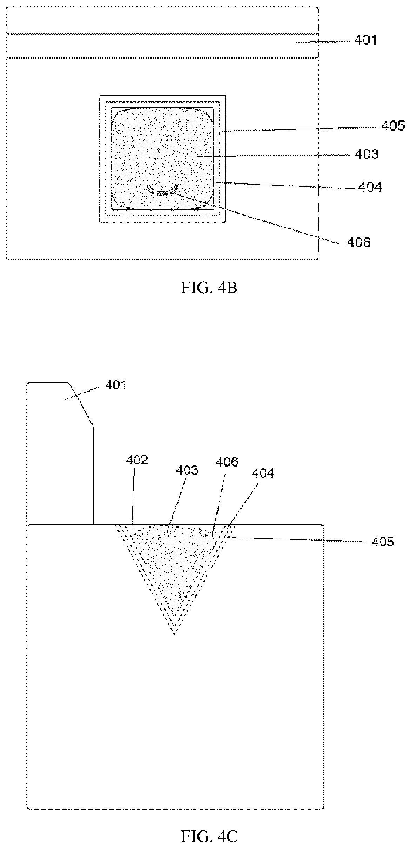

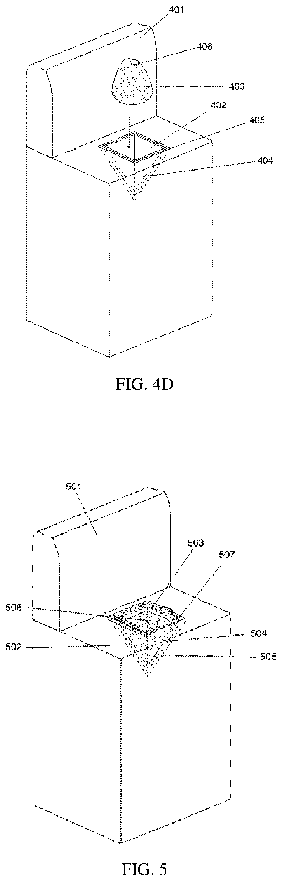

FIG.'s 4A-D illustrate a peritoneal dialysis system **401** using a silicone rubber and conductive plate heater in a triangular receiving compartment **402**. FIG.4A is a perspective view of the peritoneal dialysis system **401**, FIG. 4B is a top view of the peritoneal dialysis system **401**, FIG. 4C is a side view of peritoneal dialysis system **401**, and FIG. 4D shows a fluid bag **403** being placed into the triangular receiving compartment **402**. As illustrated in FIG. 4A, the peritoneal dialysis system can include a triangular receiving compartment **402** into which the fluid bag **303** can be placed for heating. The heater illustrated in FIG.'s 4A-D is similar to that of FIG.'s 2A-D but with a triangular rather than rectangular receiving compartment. The heater can include a first layer of silicone rubber **405** and a conductive plate **404** positioned on top of the silicone rubber **405**. The layer of silicone rubber **405** has embedded heating elements (not shown) that heat up in response to an electrical current to heat fluid in the fluid bag **403**. As described, the fluid bag **403** can include a handle **406** for easier moving of the fluid bag **403**.

As illustrated in FIG. 4B, the dialysis fluid bag **403** can fit entirely within the peritoneal dialysis system **401**. Once placed inside, the fluid bag **403** can be heated by the silicone rubber **405** and conductive plate **404**. As illustrated in FIG. 4C, the fluid bag **403** can be placed fully within the receiving compartment **402**. The silicone rubber **405** and conductive plate **404** can line all or part of the inner surface of the receiving compartment **402**, contacting the fluid bag **403**. As illustrated in FIG. 4D, the fluid bag **403** can be loaded into the receiving compartment **402** from the top of the peritoneal dialysis system **401.** However, as described, any other arrangement can be used for the receiving compartment **402.**

FIG. 5 illustrates a peritoneal dialysis system **501** having a triangular receiving compartment **502,** similar to that shown in FIG.'s 4A-D. A layer of silicone rubber **505** and a conductive plate **504** can line the inner surface of receiving compartment **502** to heat fluid in fluid bag **503.** As described, the fluid bag **503** can include handle **506** for easier movement. The embodiment shown in FIG. 5 includes a lid **507** covering the receiving compartment **502** allowing the option to enclose the fluid bag **503** in the receiving compartment for heating. The lid **507** can also be lined with a layer of silicone rubber and conductive plate.

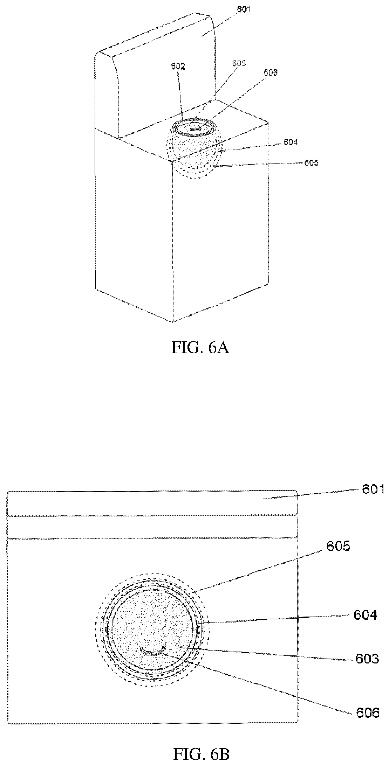

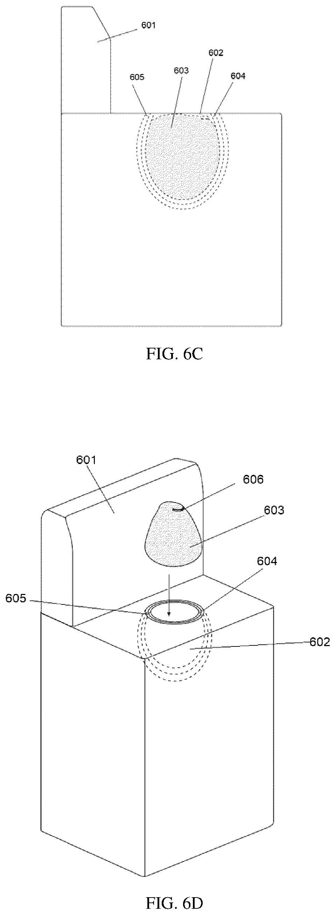

FIG.'s 6A-D illustrate a peritoneal dialysis system **601** using a silicone rubber heater in a substantially round receiving compartment **602.** FIG.6A is a perspective view of the peritoneal dialysis system **601,** FIG. 6B is a top view of the peritoneal dialysis system **601,** FIG. 6C is a side view of peritoneal dialysis system **601,** and FIG. 6D shows a fluid bag **603** being placed into the heater. As illustrated in FIG. 6A, the peritoneal dialysis system **601** can include a round receiving compartment **602** into which the fluid bag **603** can be placed for heating. The heater illustrated in FIG.'s 6A-D is similar to that of FIG.'s 2A-D but with a round rather than rectangular receiving compartment **602.** The heater can include a first layer of silicone rubber **605** and a conductive plate **604** positioned on top of the silicone rubber **604.** The layer of silicone rubber **605** has embedded heating elements (not shown) that heat up in response to an electrical current to heat fluid in the fluid bag **603.** As described, the fluid bag **603** can include a handle **606** for easier moving of the fluid bag **603.**

As illustrated in FIG. 6B, the dialysis fluid bag **603** can fit entirely within the peritoneal dialysis system **601.** Once placed inside, the fluid bag **603** can be heated by the silicone rubber **605** and conductive plate **604.** As illustrated in FIG. 6C, the fluid bag **603** can be placed fully within the receiving compartment **602.** The silicone rubber **605** and conductive plate **604** can line the inner surface of the receiving compartment **602,** contacting the fluid bag **603.** As illustrated in FIG. 6D, the fluid bag **603** can be loaded into the receiving compartment **602** from the top of the peritoneal dialysis system **601.** However, as described, any other arrangement can be used for the receiving compartment **602.**

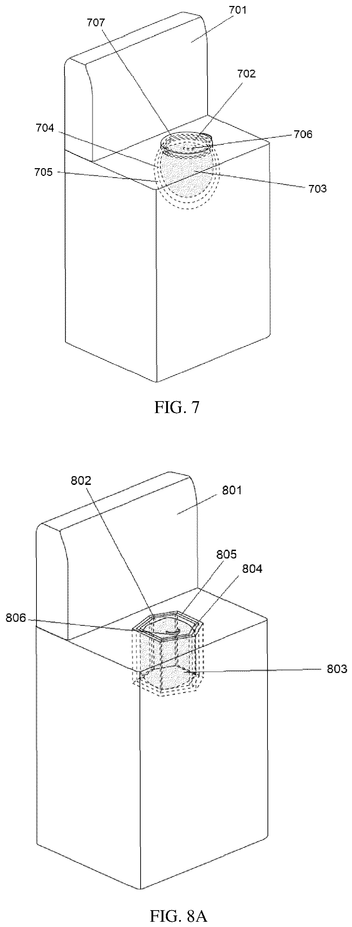

FIG. 7 illustrates a peritoneal dialysis system **701** having a substantially round receiving compartment **702,** similar to that shown in FIG.'s 6A-D. A layer of silicone rubber **705** and a conductive plate **704** can line the inner surface of receiving compartment **702** to heat fluid in fluid bag **703.** As described, the fluid bag **703** can include handle **706** for easier movement. The embodiment shown in FIG. 7 includes a lid **707** covering the receiving compartment **702** allowing the option to enclose the fluid bag **703** in the receiving compartment for heating. The lid **707** can also be lined with a layer of silicone rubber and conductive plate.

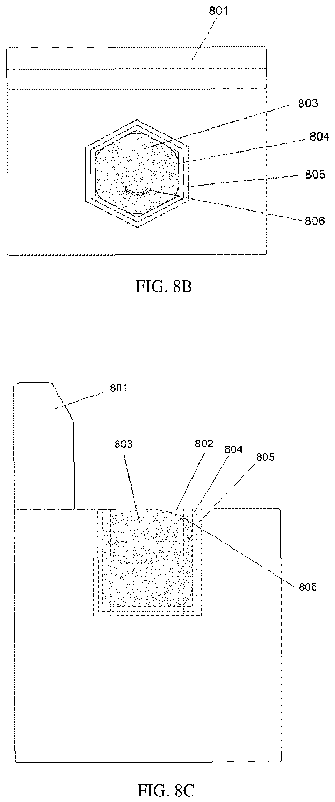

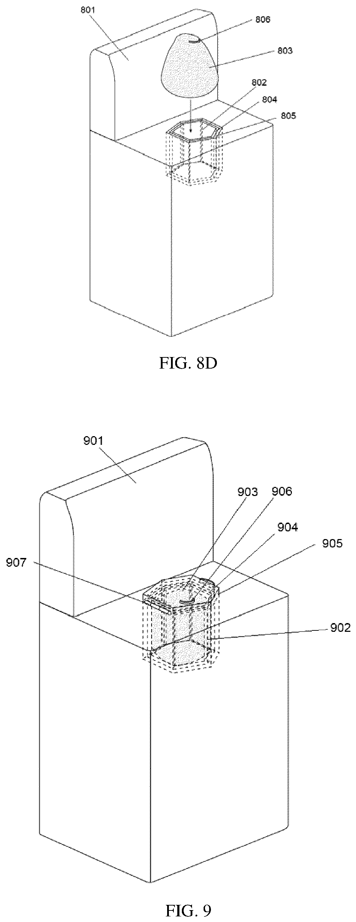

FIG.'s 8A-D illustrate a peritoneal dialysis system **801** using a silicone rubber heater in a hexagonal receiving compartment **802.** Although shown as hexagonal receiving compartment **802** in FIG.'s 8A-D, one of skill in the art will understand that any shape can be used for a receiving compartment. FIG. 8A is a perspective view of the peritoneal dialysis system **801,** FIG. 8B is a top view of the peritoneal dialysis system **801,** FIG. 8C is a side view of peritoneal dialysis system **801,** and FIG. 8D shows a fluid bag **803** being placed into the heater. As illustrated in FIG. 8A, the peritoneal dialysis system can include any shape receiving compartment, such as hexagonal receiving compartment **802** into which the fluid bag **803** can be placed for heating. The heater can include a first layer of silicone rubber **805** and a conductive plate **804** positioned on top of the silicone rubber **804.** The layer of silicone rubber **805** has embedded heating elements (not shown) that heat up in response to an electrical current to heat fluid in the fluid bag **803.** As described, the fluid bag **803** can include a handle **806** for easier moving of the fluid bag **803.**

As illustrated in FIG. 8B, the dialysis fluid bag **803** can fit entirely within the peritoneal dialysis system **801.** Once placed inside, the fluid bag **803** can be heated by the silicone rubber **805** and conductive plate **804.** As illustrated in FIG. 8C, the fluid bag **803** can be placed fully within the receiving compartment **802.** The silicone rubber **805** and conductive plate **804** can line the inner surface of the receiving compartment **802,** contacting the fluid bag **803.** As illustrated in FIG. 8D, the fluid bag **803** can be loaded into the receiving compartment **802** from the top of the peritoneal dialysis system **801.** However, as described, any other arrangement can be used for the receiving compartment **802.**

FIG. 9 illustrates a peritoneal dialysis system **901** having a hexagonal receiving compartment **902,** similar to that shown in FIG.'s 8A-D. A layer of silicone rubber **905** and a conductive plate **904** can line the inner surface of receiving compartment **902** to heat fluid in fluid bag **903.** As described, the fluid bag **903** can include handle **906** for easier movement. The embodiment shown in FIG. 9 includes a lid **907** covering the receiving compartment **902** allowing the option to enclose the fluid bag **903** in the receiving compartment for heating. The lid **907** can also be lined with a layer of silicone rubber and conductive plate.

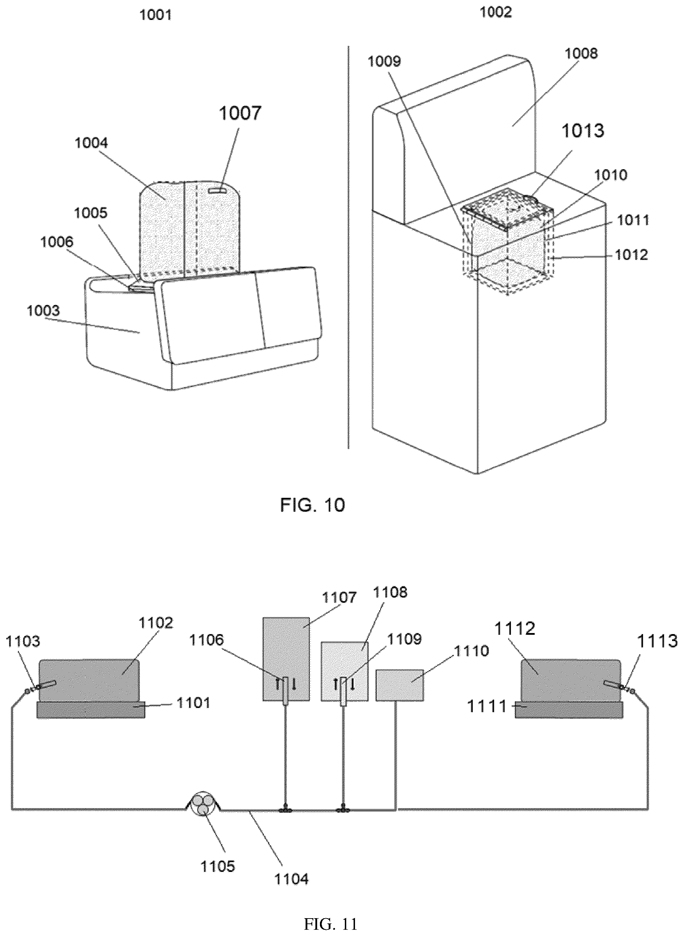

FIG. 10 illustrates a peritoneal dialysis system using two heaters of the invention. The system can include a peritoneal dialysis fluid generation module **1008** and a peritoneal dialysis cycler **1003.** The peritoneal dialysis fluid generation module **1008** is used to generate peritoneal dialysis fluid from solid or concentrated components. The peritoneal dialysis cycler **1003** is used to infuse peritoneal dialysis fluid into a patient, and optionally to drain used peritoneal dialysis fluid from the patient. To generate peritoneal dialysis fluid from solid material, the solid material must first be dissolved. A first heater can be used to accurately and precisely heat a source water to a desired temperature to dissolve a solid material, speeding dissolution. The heater can be quickly adjusted to a second temperatures to dissolve a second solid material having different dissolution properties than the first material. As illustrated in FIG. 10, the peritoneal dialysis fluid generation module **1008** can include a receiving compartment **1009** into which a fluid bag **1010** can be placed. The fluid bag **1010** can contain water to be used in dissolving solid components of the peritoneal dialysis fluid. The heater can include a first layer of silicone rubber **1012** with embedded heating elements. A conductive plate **1011** can be positioned on top of the silicone rubber **1012.** As described, the heater can heat the fluid in fluid bag **1010** to a desired temperature. The heated fluid can then be pumped into one or more concentrate sources to dissolve the material, which can then be mixed and diluted to generate peritoneal dialysis fluid. The receiving compartment **1009** can optionally include a lid **1013,** as described.

Concentrates can be mixed and added into a second fluid bag **1004.** In certain embodiments, the concentrates can be individually added to fluid bag **1004** and mixed within the fluid bag **1004.** Alternatively, the concentrates can be added to a separate mixing bag or container and the final peritoneal dialysis fluid added to fluid bag **1004.** Each concentrate can have a different dissolution profile requiring a different fluid or water temperature. The heater of the invention can quickly and accurately and/or precisely heat the fluid or water to the desired temperature for the specific concentrate.

As illustrated in FIG. 10, the fluid bag **1004** can be placed on top of a silicone rubber heater on a peritoneal dialysis cycler **1003.** The heater can include silicone rubber **1006** with embedded heating elements (not shown) and conductive plate **1005** positioned on top of the silicone rubber **1006.** The peritoneal dialysis fluid in fluid bag **1004** can be heated to a desired temperature and then infused into the peritoneal cavity of the patient through any means known in the art. In certain embodiments, fluid bag **1004** can include handle **1007** for ease of movement.

As illustrated in FIG. 10, the system can include a separate peritoneal dialysis fluid generation module **1008** and peritoneal dialysis cycler **1003.** Two separate heaters can be employed, with one heater for each of the peritoneal dialysis fluid generation module **1008** and peritoneal dialysis cycler **1003.** Alternatively, a single module that combines the peritoneal dialysis fluid generation module **1008** and peritoneal dialysis cycler **1003** can be used with a single or multiple heater. Using a separate peritoneal dialysis fluid generation module **1008** and peritoneal dialysis cycler **1003** can enable the components to be spaced apart. For example, the peritoneal dialysis fluid generation module **1008** can be placed in a first room **1002** near to a water supply (not shown). The peritoneal dialysis cycler **1003** can be placed in a separate room **1001** where treatment is more convenient. The user can move the fluid bag **1004** containing the final peritoneal dialysis fluid from the peritoneal dialysis fluid generation module **1008** to the peritoneal dialysis cycler **1003.** Alternatively, a tube or hose can carry the peritoneal dialysis fluid into the fluid bag **1004** where the fluid is heated prior to treatment.

Although the heaters illustrated in FIG. 10 are shown as being in a receiving compartment **1009** on the peritoneal dialysis fluid generation module **1008** and on a substantially flat surface on the peritoneal dialysis cycler **1003,** one of skill in the art will understand that any arrangement can be used for either module. For example, both modules can use receiving compartments, both modules can use substantially flat surfaces, or any combination can be used.

FIG. 11 illustrates the use of a silicone rubber heater **1101** in a peritoneal dialysis fluid generation module. A fluid bag **1102,** which can contain purified water, can be placed on the silicone rubber heater **1101.** A controller (not shown) can control the silicone rubber heater **1101** to heat the water in fluid bag **1102** to a desired temperature. Pump **1105** can pump the heated water through an outlet **1103** of fluid bag **1102** into fluid line **1104.** The system can include valves and/or additional pumps (not shown) to control the movement of fluid. Purified water can be pumped into a first concentrate source **1107** through connector **1106,** into second concentrate source **1108** through connector **1109,** and into a third concentrate source **1110.** Solid material initially contained in each concentrate source can be dissolved with the water. The concentrates thus generated can then be pumped through fluid line **1104** into a second fluid bag **1112** through inlet **1113** to generate the final peritoneal dialysis fluid. Silicone rubber heater **1111** can heat the final peritoneal dialysis fluid prior to infusion into the patient. As described, in certain embodiments silicone rubber heater **1111** can be part of a separate peritoneal dialysis cycler and need not be positioned as part of the peritoneal dialysis fluid generation module.

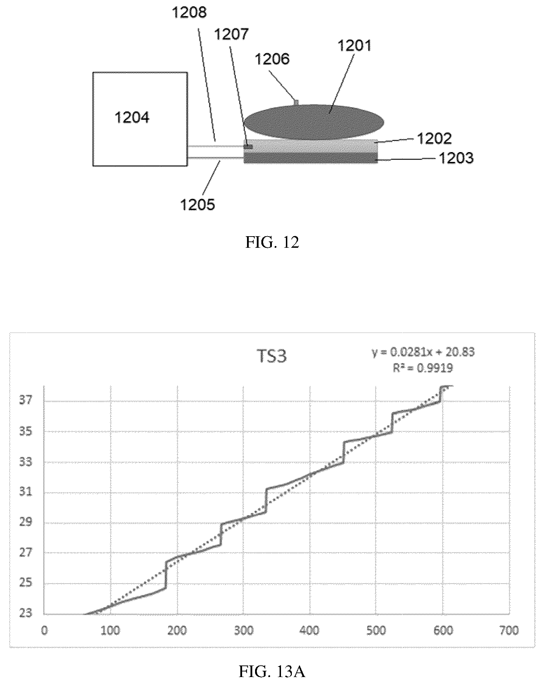

FIG. 12 illustrates an experimental setup to test the silicone rubber heater. An EZ-zone watlow® heater controller **1204** was used to control heating elements embedded in a layer of silicone rubber **1203.** The heater controller **1204** was connected to a PT100 temperature sensor **1207** in conductive plate **1202** by wire **1208.** A fluid bag **1201** was placed on top of the conductive plate **1202.** A second PT100 temperature sensor **1206** was connected to the fluid bag **1201** to measure the temperature of the fluid. 3 L of peritoneal dialysis fluid at 25°C was placed inside fluid bag **1201.** The temperature of the silicone rubber was set to 60°C. A timer was started and stopped when the temperature of the peritoneal dialysis fluid reached 37°C. The process was repeated with the silicone rubber heater set at each of 65°C and 70°C. The experiment gives a qualitative determination about the effectiveness of the silicone rubber heater, and in particular, whether the silicone rubber heater can heat 3 L of peritoneal dialysis fluid to 37°C $\pm$ 2°C.

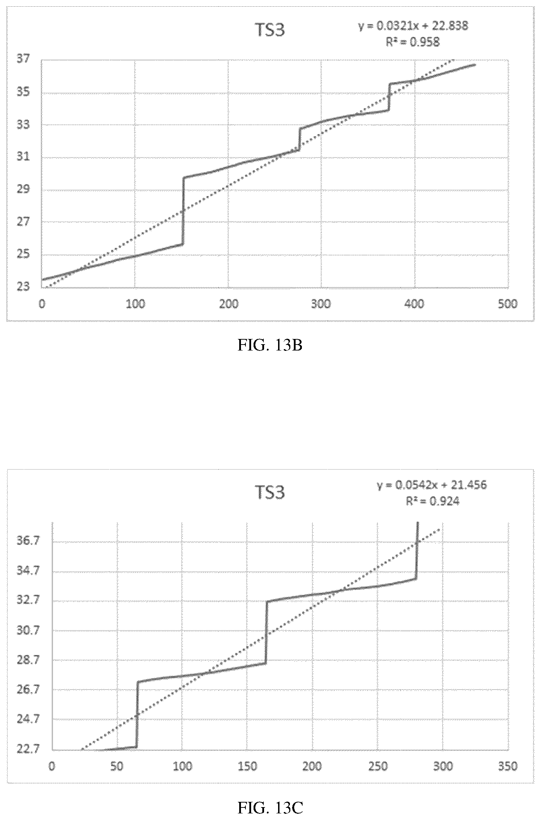

FIG.'s 13A-C are graphs of temperature vs. time for the peritoneal dialysis fluid tested with the experimental setup illustrated in FIG. 12. As illustrated in FIG. 13A, with the heater set at 60°C, the peritoneal dialysis fluid reached 37°C after 527 seconds, or 8.78 minutes. When the heater was set to 65°C, the peritoneal dialysis fluid reached 37°C after 503 seconds, or 8.38 minutes, as shown in FIG. 13B. With the heater set at 70°C, the peritoneal dialysis fluid reached 37°C after 519 seconds, or 8.65 minutes, as shown in FIG. 13C. The solid lines in FIG.'s 13A-C are the real temperature values plotted by a temperature logger at discrete intervals. The dashed lines in each of FIG.'s 13A-C are theoretical trends of the temperature increases Table 1 provides the results for each experiment.

Table 1

| Set Temperature | Change in Temperature (ΔT) | Time | α |
|---|---|---|---|
| 60°C | 13.987 °C | 8.78 min | 1.592 °C/min |
| 65°C | 13.506 °C | 8.38 min | 1.611 °C/min |
| 70°C | 14.154 °C | 8.65 min | 1.636 °C/min |

For each set temperature, Table 1 provides the total change in temperature of the peritoneal dialysis fluid, the time to reach 37°C, and $\alpha$, which is the rate of change in temperature given by $\Delta T$ divided by time. Eq(1) provides the value $\alpha$, where $\Delta T$ is the change in temperature and $\Delta t$ is the change in time. The $\Delta T$ varies slightly between the three experiments due to slight variations in starting temperature of the peritoneal dialysis fluid.

$$\alpha = \frac{\Delta T}{\Delta t} = \frac{°C}{min} \qquad\qquad Eq(1)$$

For each set point, the silicone rubber heater was able to heat the peritoneal dialysis fluid effectively to 37°C. The higher the set point used, the quicker the heater is able to heat the peritoneal dialysis fluid, given by value $\alpha$. In FIG.'s 13A-C, $\alpha$ is the angular coefficient of the ideal line, shown as the dashed line for each of FIG.'s 13A-C. As shown in Table 1, each setting resulted in heating of the 3 L of peritoneal dialysis fluid at a rate of between 15.9 and 16.4 °C/min, illustrating that the silicone rubber heater can effectively heat peritoneal dialysis fluid to a desired temperature quickly with a range of set points used with a starting temperature of between 22-23°C, or room temperature.

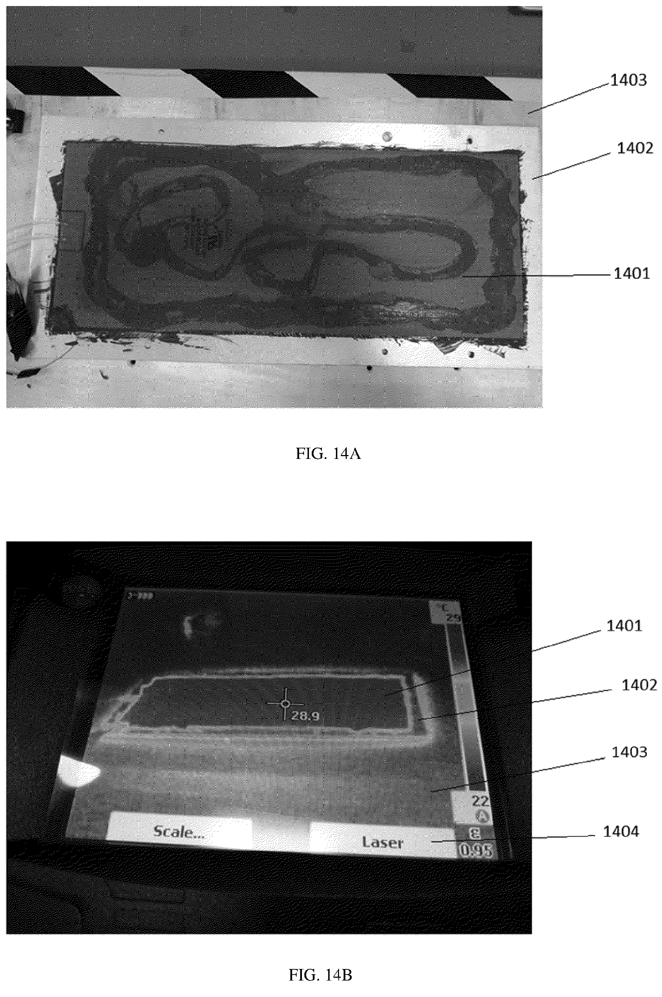

FIG.'s 14A-C illustrate an experiment to test the uniformity of heating fluid in a bag using a silicone rubber heater. FIG. 14A is a picture of a deconstructed heater. In FIG. 14A, the heater is shown upside down. A layer of silicone rubber 1401 containing one or more heating elements is shown on top of a conductive plate 1402. The silicone rubber heater was positioned on a wooden plate 1403 as a base to avoid burning the table. In certain embodiments, the conductive plate 1402 and/or layer of silicone rubber 1401 can be attached to the wooden plate 1403. Adhesives or mechanical connectors, such as screws or nails, can be inserted through the conductive plate 1402 and/or layer of silicone rubber 1401 to connect to the wooden plate. The wooden plate can avoid burning of the other components of the dialysis system.

FIG. 14B shows a display screen 1404 with a thermal image of a bag 1405 placed on a silicone rubber heater during heating. The bag 1405 is on top of conductive plate 1402. The heater is placed on a wooden plate 1403. The layer of silicone rubber is not visible in FIG. 14B. As illustrated in FIG. 14B, the bag was a nearly uniform 28.9 °C throughout. The conductive plate 1402 and wooden base 1403 were significantly cooler.

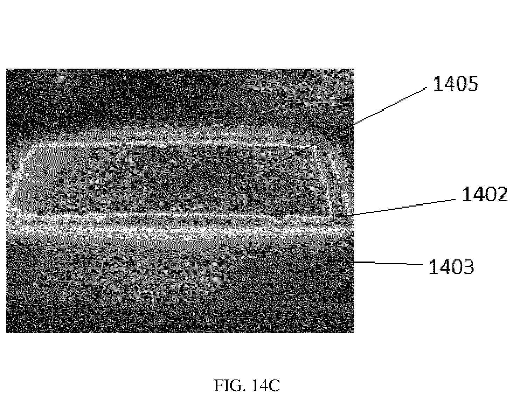

FIG. 14C is a close up of the thermal image illustrated in FIG. 14B. The bag 1405 was nearly uniform in temperature, although slight deviations occurred at the end of the bag 1405. As described, the conductive plate 1402 and wooden base 1403 were significantly cooler. As illustrated in FIG.'s 14A-C, the silicone rubber heater described provides effective heating of a fluid bag with a largely uniform temperature distribution.

One skilled in the art will understand that various combinations and/or modifications and variations can be made in the described systems and methods depending upon the specific needs for operation. Various aspects disclosed herein may be combined in different combinations than the combinations specifically presented in the description and accompanying drawings. The invention is however defined by the appended claims.

**Claims**

1. A peritoneal dialysis system (201, 301, 401, 501, 601, 701, 801, 901), comprising:

   a fluid bag (203, 303, 403, 503, 603, 703, 803, 903) containing a peritoneal dialysis fluid; and
   a peritoneal dialysis cycler (1003) comprising:
   a receiving compartment (202, 302, 402, 502, 602, 702, 802, 902) having an inner surface defining a cavity, the fluid bag being received in the cavity; and a heater comprising:

   a first layer of silicon rubber (205, 305, 405, 505, 605, 705, 805, 905) having one or more heating elements embedded therein, wherein the one or more heating elements are uniformly distributed through the first layer of silicon rubber; and
   a conductive plate (204, 304, 404, 504, 604, 704, 804, 904) positioned on a first side of the first layer of silicon rubber, wherein the area of the conductive plate is substantially similar to the area of the first side of the first layer of silicon rubber,

**characterised in that** the first layer of silicon rubber and the conductive plate line the inner surface of the receiving compartment, whereby the entire receiving compartment can be used to heat the fluid bag placed inside the receiving compartment, and the fluid bag fits entirely within the receiving compartment.

2. The system of claim 1, wherein the conductive plate (204, 304, 404, 504, 604, 704, 804, 904) is selected from any one of an aluminum plate, silver plate, copper plate, gold plate, silicon carbide plate, tungsten plate, graphite plate, zinc plate, and any combination thereof.

3. The system of claim 1 or 2, wherein the receiving compartment (202, 302) is substantially rectangular.

4. The system of claim 1 or 2, wherein the receiving compartment (602, 702) is substantially round.

5. A peritoneal dialysis system (201, 301, 401, 501, 601, 701, 801, 901), comprising:

a fluid bag (203, 303, 403, 503, 603, 703, 803, 903) containing purified water; and
a peritoneal dialysis fluid generation module (1008) comprising:
a receiving compartment (202, 302, 402, 502, 602, 702, 802, 902, 1009) having an inner surface defining a cavity, the fluid bag being received in the cavity; and; and
a heater comprising:

a first layer of silicon rubber (205, 305, 405, 505, 605, 705, 805, 905, 1012) having one or more heating elements embedded therein, wherein the one or more heating elements are uniformly distributed through the first layer of silicon rubber; and
a conductive plate (204, 304, 404, 504, 604, 704, 804, 904, 1011) positioned on a first side of the first layer of silicon rubber, wherein the area of the conductive plate is substantially similar to the area of the first side of the first layer of silicon rubber,
**characterised in that** the first layer of silicon rubber and the conductive plate line the inner surface of the receiving compartment, and the fluid bag fits entirely within the receiving compartment, whereby the entire inner surface of the receiving compartment can be used to heat the fluid bag.

6. The system of claim 5, wherein the conductive plate (204, 304, 404, 504, 604, 704, 804, 904, 1011) is selected from any one of an aluminum plate, silver plate, copper plate, gold plate, silicon carbide plate, tungsten plate, graphite plate, zinc plate, and any combination thereof.

7. The system of claim 5 or 6, wherein the receiving compartment (202, 302, 1009) is substantially rectangular.

8. The system of claim 5 or 6, wherein the receiving compartment (602, 702) is substantially round.

9. The system of any of claims 5 to 8, further comprising a peritoneal dialysis system according to any of claims 1 to 4.

10. A method, comprising the steps of:

heating purified water in the fluid bag with the heater of the peritoneal dialysis system (201, 301, 401, 501, 601, 701, 801, 901) of any of claims 5 to 9; and
generating a peritoneal dialysis fluid using the purified water.

11. The method of claim 10, further comprising the step of pumping the purified water into a concentrate source (1107, 1108, 1110) to generate a concentrate; the concentrate source initially containing a solid material.

12. The method of claim 11, further comprising the steps of pumping the purified water into at least two concentrate sources (1107, 1108, 1110) to generate at least two concentrates; the at least two concentrate sources each initially containing a solid material.

# EP 4 019 061 B1

**Patentansprüche**

1. Peritonealdialysesystem (201, 301, 401, 501, 601, 701, 801, 901), umfassend:

   einen Fluidbeutel (203, 303, 403, 503, 603, 703, 803, 903), der ein Peritonealdialysefluid enthält; und
   einen Peritonealdialysezyklusregler (1003), umfassend:
   ein Aufnahmefach (202, 302, 402, 502, 602, 702, 802, 902), das eine Innenoberfläche aufweist, die einen Hohlraum definiert, wobei der Fluidbeutel in dem Hohlraum aufgenommen ist; und eine Heizeinrichtung, umfassend:

   eine erste Schicht aus Silikonkautschuk (205, 305, 405, 505, 605, 705, 805, 905), die ein oder mehrere darin eingebettete Heizelemente aufweist, wobei das eine oder die mehreren Heizelemente gleichmäßig durchgehend durch die erste Schicht aus Silikonkautschuk verteilt sind; und
   eine leitfähige Platte (204, 304, 404, 504, 604, 704, 804, 904), die an einer ersten Seite der ersten Schicht aus Silikonkautschuk positioniert ist, wobei die Fläche der leitfähigen Platte der Fläche der ersten Seite der ersten Schicht aus Silikonkautschuk im Wesentlichen ähnlich ist,
   **dadurch gekennzeichnet, dass** die erste Schicht aus Silikonkautschuk und die leitfähige Platte die Innenoberfläche des Aufnahmefachs auskleiden, wodurch das gesamte Aufnahmefach verwendet werden kann, um den Fluidbeutel, der innerhalb des Aufnahmefachs platziert ist, zu erhitzen, und der Fluidbeutel vollständig in das Aufnahmefach passt.

2. System nach Anspruch 1, wobei die leitfähige Platte (204, 304, 404, 504, 604, 704, 804, 904) aus einer beliebigen von einer Aluminiumplatte, Silberplatte, Kupferplatte, Goldplatte, Siliziumkarbidplatte, Wolframplatte, Graphitplatte, Zinkplatte und einer beliebigen Kombination davon ausgewählt ist.

3. System nach Anspruch 1 oder 2, wobei das Aufnahmefach (202, 302) im Wesentlichen rechteckig ist.

4. System nach Anspruch 1 oder 2, wobei das Aufnahmefach (602, 702) im Wesentlichen rund ist.

5. Peritonealdialysesystem (201, 301, 401, 501, 601, 701, 801, 901), umfassend:

   einen Fluidbeutel (203, 303, 403, 503, 603, 703, 803, 903), der gereinigtes Wasser enthält; und
   ein Modul (1008) zum Erzeugen von Peritonealdialysefluid, umfassend:

   ein Aufnahmefach (202, 302, 402, 502, 602, 702, 802, 902, 1009), das eine Innenoberfläche aufweist, die einen Hohlraum definiert, wobei der Fluidbeutel in dem Hohlraum aufgenommen ist; und; und
   eine Heizeinrichtung, umfassend:

   eine erste Schicht aus Silikonkautschuk (205, 305, 405, 505, 605, 705, 805, 905, 1012), die ein oder mehrere darin eingebettete Heizelemente aufweist, wobei das eine oder die mehreren Heizelemente gleichmäßig durchgehend durch die erste Schicht aus Silikonkautschuk verteilt sind; und
   eine leitfähige Platte (204, 304, 404, 504, 604, 704, 804, 904, 1011), die an einer ersten Seite der ersten Schicht aus Silikonkautschuk positioniert ist, wobei die Fläche der leitfähigen Platte der Fläche der ersten Seite der ersten Schicht aus Silikonkautschuk im Wesentlichen ähnlich ist,
   **dadurch gekennzeichnet, dass** die erste Schicht aus Silikonkautschuk und die leitfähige Platte die Innenoberfläche des Aufnahmefachs auskleiden, und der Fluidbeutel vollständig in das Aufnahmefach passt, wodurch die gesamte Innenoberfläche des Aufnahmefachs verwendet werden kann, um den Fluidbeutel zu erhitzen.

6. System nach Anspruch 5, wobei die leitfähige Platte (204, 304, 404, 504, 604, 704, 804, 904, 1011) aus einer beliebigen von einer Aluminiumplatte, Silberplatte, Kupferplatte, Goldplatte, Siliziumkarbidplatte, Wolframplatte, Graphitplatte, Zinkplatte und einer beliebigen Kombination davon ausgewählt ist.

7. System nach Anspruch 5 oder 6, wobei das Aufnahmefach (202, 302, 1009) im Wesentlichen rechteckig ist.

8. System nach Anspruch 5 oder 6, wobei das Aufnahmefach (602, 702) im Wesentlichen rund ist.

9. System nach einem der Ansprüche 5 bis 8, ferner umfassend ein Peritonealdialysesystem nach einem der Ansprüche 1 bis 4.

10. Verfahren, umfassend die Schritte:

Erhitzen von gereinigtem Wasser in dem Fluidbeutel mit der Heizeinrichtung des Peritonealdialysesystems (201, 301, 401, 501, 601, 701, 801, 901) nach einem der Ansprüche 5 bis 9; und
Erzeugen eines Peritonealdialysefluids unter Verwendung des gereinigten Wassers.

11. Verfahren nach Anspruch 10, ferner umfassend den Schritt eines Pumpens des gereinigten Wassers in eine Konzentratquelle (1107, 1108, 1110), um ein Konzentrat zu erzeugen; die Konzentratquelle anfänglich ein festes Material enthält.

12. Verfahren nach Anspruch 11, ferner umfassend die Schritte des Pumpens des gereinigten Wassers in mindestens zwei Konzentratquellen (1107, 1108, 1110), um mindestens zwei Konzentrate zu erzeugen; wobei die mindestens zwei Konzentratquellen jeweils anfänglich ein festes Material enthalten.

**Revendications**

1. Système de dialyse péritonéale (201, 301, 401, 501, 601, 701, 801, 901), comprenant :

une poche de liquide (203, 303, 403, 503, 603, 703, 803, 903) contenant un fluide de dialyse péritonéale ; et
un cycleur de dialyse péritonéale (1003), comprenant :
un compartiment de réception (202, 302, 402, 502, 602, 702, 802, 902) présentant une surface interne définissant une cavité, la poche de fluide étant logée dans la cavité ; et un dispositif de chauffage comprenant :

une première couche de caoutchouc de silicone (205, 305, 405, 505, 605, 705, 805, 905) dans laquelle sont intégrés un ou plusieurs éléments chauffants, dans lequel le ou les éléments chauffants sont uniformément répartis à travers la première couche de caoutchouc de silicone ; et
une plaque conductrice (204, 304, 404, 504, 604, 704, 804, 904) positionnée sur une première face de la première couche de caoutchouc de silicone, dans lequel l'aire de la plaque conductrice est sensiblement similaire à l'aire de la première face de la première couche de caoutchouc de silicone,
**caractérisé en ce que** la première couche de caoutchouc de silicone et la plaque conductrice tapissent la surface interne du compartiment de réception, moyennant quoi tout le compartiment de réception peut être utilisé pour le chauffage de la poche de fluide placée à l'intérieur du compartiment de réception et la poche de fluide s'insère entièrement à l'intérieur du compartiment de réception.

2. Système selon la revendication 1, dans lequel la plaque conductrice (204, 304, 404, 504, 604, 704, 804, 904) est choisie parmi l'une quelconque parmi une plaque d'aluminium, une plaque d'argent, une plaque de cuivre, une plaque d'or, une plaque de carbure de silicium, une plaque de tungstène, une plaque de graphite, une plaque de zinc et une quelconque combinaison de celles-ci.

3. Système selon la revendication 1 ou 2, dans lequel le compartiment de réception (202, 302) est sensiblement rectangulaire.

4. Système selon la revendication 1 ou 2, dans lequel le compartiment de réception (602, 702) est sensiblement rond.

5. Système de dialyse péritonéale (201, 301, 401, 501, 601, 701, 801, 901), comprenant :

une poche de fluide (203, 303, 403, 503, 603, 703, 803, 903) contenant de l'eau purifiée ; et
un module de génération de fluide de dialyse péritonéale (1008) comprenant :

un compartiment de réception (202, 302, 402, 502, 602, 702, 802, 902, 1009) présentant une surface interne définissant une cavité, la poche de fluide étant logée dans la cavité ; et ; et
un dispositif de chauffage comprenant :

une première couche de caoutchouc de silicone (205, 305, 405, 505, 605, 705, 805, 905, 1012) dans laquelle sont intégrés un ou plusieurs éléments chauffants, dans lequel le ou les éléments chauffants sont uniformément répartis à travers la première couche de caoutchouc de silicone ; et

une plaque conductrice (204, 304, 404, 504, 604, 704, 804, 904, 1011) positionnée sur une première face de la première couche de caoutchouc de silicone, dans lequel l'aire de la plaque conductrice est sensiblement similaire à l'aire de la première face de la première couche de caoutchouc de silicone, **caractérisé en ce que** la première couche de caoutchouc de silicone et la plaque conductrice tapissent la surface interne du compartiment de réception et la poche de fluide s'insère entièrement dans le compartiment de réception, moyennant quoi toute la surface interne du compartiment de réception peut être utilisée pour le chauffage de la poche de fluide.

6. Système selon la revendication 5, dans lequel la plaque conductrice (204, 304, 404, 504, 604, 704, 804, 904, 1011) est choisie parmi l'une quelconque parmi une plaque d'aluminium, une plaque d'argent, une plaque de cuivre, une plaque d'or, une plaque de carbure de silicium, une plaque de tungstène, une plaque de graphite, une plaque de zinc et une quelconque combinaison de celles-ci.

7. Système selon la revendication 5 ou 6, dans lequel le compartiment de réception (202, 302, 1009) est sensiblement rectangulaire.

8. Système selon la revendication 5 ou 6, dans lequel le compartiment de réception (602, 702) est sensiblement rond.

9. Système selon l'une quelconque des revendications 5 à 8, comprenant en outre un système de dialyse péritonéale selon l'une quelconque des revendications 1 à 4.

10. Procédé, comprenant les étapes consistant à :

chauffer de l'eau purifiée dans la poche de fluide à l'aide du dispositif de chauffage du système de dialyse péritonéale (201, 301, 401, 501, 601, 701, 801, 901) selon l'une quelconque des revendications 5 à 9 ; et générer un fluide de dialyse péritonéale à l'aide de l'eau purifiée.

11. Procédé selon la revendication 10, comprenant en outre l'étape consistant à pomper l'eau purifiée dans une source de concentré (1107, 1108, 1110) afin de générer un concentré ; la source de concentré contenant initialement un matériau solide.

12. Procédé selon la revendication 11, comprenant en outre les étapes consistant à pomper l'eau purifiée dans au moins deux sources de concentré (1107, 1108, 1110) afin de générer au moins deux concentrés ; les au moins deux sources de concentré contenant chacune initialement un matériau solide.

FIG. 1A

FIG. 1B

FIG. 1C

FIG. 1D

FIG. 2A

FIG. 2B

FIG. 2C

FIG. 2D

FIG. 3

FIG. 4A

401

405

403

404

406

FIG. 4B

401

402　403　406　404

405

FIG. 4C

FIG. 4D

FIG. 5

FIG. 6A

FIG. 6B

FIG. 6C

FIG. 6D

FIG. 7

FIG. 8A

FIG. 8B

FIG. 8C

FIG. 8D

FIG. 9

1001

1002

1009

1008

1007

1004

1013

1005

1010

1006

1011

1003

1012

FIG. 10

1107 1108

1102

1106

1109

1103

1112

1113

1110

1101

1111

1105

1104

FIG. 11

EP 4 019 061 B1

FIG. 12

FIG. 13A

FIG. 13B

FIG. 13C

FIG. 14A

FIG. 14B

FIG. 14C

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 20140199057 A1